# EUROPEAN PATENT APPLICATION

(11) **EP 4 053 154 A1**
(43) Date of publication of application: **07.09.2022**
(21) Application number: 20881088.7
(22) Date of filing: 28.10.2020
(51) Int. Cl.: C07K 14/47, C12N 15/12, C12N 15/63, A61K 38/17, A61P 11/00, A61P 29/00, A61P 25/08, A61P 31/12, A61P 9/12

(54) **KERATIN BD-10, PREPARATION METHOD THEREFOR, AND PHARMACEUTICAL COMPOSITION THEREOF, AND USE THEREOF**

(30) Priority: 28.10.2019 CN 201911028718
(71) Applicant: INSTITUTE OF MATERIA MEDICA, CHINESE ACADEMY OF MEDICAL SCIENCES, Beijing 100050 (CN)
(72) Inventor: YU, Shishan, Beijing 100050 (CN); WANG, Xiaoliang, Beijing 100050 (CN); FU, Jiang, Beijing 100050 (CN); LIU, Yunbao, Beijing 100050 (CN); FENG, Nan, Beijing 100050 (CN); ZHANG, Mi, Beijing 100050 (CN); SHI, Guoru, Beijing 100050 (CN); CAI, Jie, Beijing 100050 (CN)
(74) Representative: V.O.
(86) International application number: PCT/CN2020/124359
(87) International publication number: WO 2021/083201

(57) **Abstract**

The present invention provides a keratin BD-10, an encoding nucleic acid molecule, an expression vector, a host cell thereof, a preparation method for BD-10, and a pharmaceutical composition containing the keratin BD-10. Also provides the use of the keratin BD-10, nucleic acid molecule, expression vector, host cell or pharmaceutical composition in the preparation of medicament for antipyretic, analgesic, antitussive, expectorant, anticonvulsant, antiepileptic, anti-hypertension, anti-inflammatory, and antiviral.

SEQUENCE LISTING

<110>  Institute of Materia Medica, Chinese Academy of Medical Sciences

<120>  KERATIN BD-10, PREPARATION METHOD THEREFOR AND PHARMACEUTICAL
COMPOSITION AND USE THEREOF

<160>  2

<170>  SIPOSequenceListing 1.0

<210>  1
<211>  507
<212>  PRT
<213>  Bos taurus

<400>  1
Met Ser Cys Arg Ser Tyr Arg Ile Ser Pro Gly Tyr Ser Val Thr Arg
1               5                   10                  15
Thr Phe Ser Ser Cys Ser Ala Val Ala Pro Lys Thr Gly Ser Arg Cys
            20                  25                  30
Cys Ile Thr Ala Ala Pro Tyr Arg Gly Val Ser Cys Tyr Arg Gly Leu
        35                  40                  45
Thr Gly Phe Gly Ser Arg Ser Val Ser Ala Leu Gly Ser Cys Gly Pro
    50                  55                  60
Arg Ile Ala Val Gly Gly Phe Arg Ala Gly Ser Cys Asn Arg Ser Phe
65                  70                  75                  80
Gly Tyr Arg Ser Gly Gly Val Gly Gly Leu Ser Pro Pro Cys Ile Thr
                85                  90                  95
Thr Val Ser Val Asn Glu Ser Leu Leu Thr Pro Leu Asn Leu Glu Ile
            100                 105                 110
Asp Pro Asn Ala Gln Cys Val Lys His Glu Glu Lys Glu Gln Ile Lys
        115                 120                 125
Asn Leu Asn Ser Arg Phe Ala Ala Phe Ile Asp Lys Val Arg Phe Leu
    130                 135                 140
Glu Gln Gln Asn Lys Leu Leu Glu Thr Lys Trp Gln Phe Tyr Gln Asn
145                 150                 155                 160
Gln Arg Cys Cys Glu Ser Asn Leu Glu Pro Leu Phe Asn Gly Tyr Ile
                165                 170                 175
Glu Thr Leu Arg Arg Glu Ala Glu His Val Glu Ala Asp Gly Gly Arg
            180                 185                 190
Leu Ala Ser Glu Leu Asn His Val Gln Glu Val Leu Glu Gly Tyr Lys
        195                 200                 205
Lys Lys Tyr Glu Glu Glu Val Ala Leu Arg Ala Thr Ala Glu Asn Glu
    210                 215                 220
Phe Val Val Leu Lys Lys Asp Val Asp Cys Ala Tyr Leu Arg Lys Ser
225                 230                 235                 240
Asp Leu Glu Ala Asn Val Glu Ala Leu Val Glu Glu Ser Asn Phe Leu
                245                 250                 255
Lys Arg Leu Tyr Asp Glu Glu Ile Gln Ile Leu Asn Ala His Ile Ser
            260                 265                 270
Asp Thr Ser Val Ile Val Lys Met Asp Asn Ser Arg Asp Leu Asn Met
        275                 280                 285
Asp Cys Val Val Ala Glu Ile Lys Ala Gln Tyr Asp Asp Ile Ala Ser
    290                 295                 300
Arg Ser Arg Ala Glu Ala Glu Ser Trp Tyr Arg Asn Lys Cys Glu Glu
305                 310                 315                 320
Met Lys Ala Thr Val Ile Arg His Gly Glu Thr Leu Arg Arg Thr Lys
                325                 330                 335
Glu Glu Ile Asn Glu Leu Asn Arg Ile Ile Gln Arg Leu Thr Ala Glu
            340                 345                 350
Ile Glu Asn Ala Lys Cys Gln Arg Thr Lys Leu Glu Ala Ala Val Ala
        355                 360                 365

Glu Ala Glu Gln Gln Gly Glu Ala Ala Leu Asn Asp Ala Arg Cys Lys
    370                 375                 380
Leu Ala Gly Leu Glu Glu Ala Leu Gln Lys Ala Lys Gln Asp Met Ala
385                 390                 395                 400
Cys Leu Leu Lys Glu Tyr Gln Glu Val Met Asn Ser Lys Leu Gly Leu
                405                 410                 415
Asp Ile Glu Ile Ala Thr Tyr Arg Arg Leu Leu Glu Gly Glu Glu Gln
            420                 425                 430
Arg Leu Cys Glu Gly Val Gly Ser Val Asn Val Cys Val Ser Ser Ser
        435                 440                 445
Arg Gly Gly Val Thr Cys Gly Gly Leu Thr Tyr Ser Thr Thr Ala Gly
    450                 455                 460
Arg Gln Ile Ala Ser Gly Pro Val Ala Thr Gly Gly Ser Ile Thr Val
465                 470                 475                 480
Leu Ala Pro Asp Ser Cys Val Pro Cys Gln Pro Arg Ala Ser Ser Phe
                485                 490                 495
Ser Cys Gly Ser Ser Arg Ser Val Arg Phe Ala
            500                 505

<210>  2
<211>  1524
<212>  DNA
<213>  Bos taurus

<400>  2
atgtcgtgtc gcagttatcg catttctccg ggctactcgg tgactcggac attttcttcc      60
tgctccgcag tggcgccgaa gaccgggtca cgttgctgca ttaccgcagc cccataccgc     120
ggcgtttcgt gttatcgggg gctgaccggt tttggcagcc gcagcgtaag cgcgctgggc     180
tcttgcgggc cccgtatcgc cgtagggggc tttcgtgcgg gttcgtgtaa ccgttccttc     240
gggtatcgct cgggcggtgt cgggggatta agtcccccgt gcataacgac cgtttccgta     300
aacgaatctc tgttgacccc actgaacctt gagatcgacc ccaatgcgca gtgcgtaaaa     360
cacgaggaga aggagcagat aaagaactta aattcccgtt tcgccgcatt catcgacaag     420
gtacgcttcc tggaacagca gaataagttg ttggagacca aatggcagtt ctatcaaaac     480
caacggtgct gcgagtcgaa cttagagccc cttttcaacg ggtacatcga gacgttacgg     540
cgcgaagcag agcatgtgga ggcagatggt ggtcgtctgg cttcggagtt aaatcatgtc     600
caggaggtgt tggaagggta caaaaagaaa tatgaggaag aggtggcctt gcgtgccacc     660
gcagaaaacg agtttgtggt tctgaaaaag gatgtggatt gcgcttattt gcggaagtct     720
gatctggaag ccaacgtcga ggcattggtc gaagagtcaa acttcctgaa gcgtctgtac     780
gatgaggaga tccaaattct taacgctcat atcagcgaca cttccgtcat cgtcaagatg     840
gacaactccc gcgatttgaa catggactgt gtagtggccg aaatcaaggc ccagtacgac     900
gacattgcgt cgcgcagtcg cgcggaagcg gaatcctggt atcggaacaa atgtgaagag     960
atgaaggcaa cggtcattcg ccacggtgaa acgcttcgcc ggactaagga ggaaataaac    1020
gagcttaacc ggataatcca gcgcctgacc gcggagattg agaacgccaa gtgtcaacgg    1080
acgaagcttg aggcagcggt tgccgaggct gagcagcagg gggaagctgc gctgaacgac    1140
gcgcggtgca agttggcggg attagaagaa gcgctgcaaa aggctaagca ggatatggcc    1200
tgtctgctga aagagtacca agaagtgatg aactcaaaac ttgggctgga tatcgagatc    1260
gcgacgtacc gtcgccttct tgaaggggaa gagcagcggc tgtgtgaggg cgtgggatct    1320
gtcaatgtgt gcgtgtctag ctcgcggggc ggcgtgactt gcggaggctt gacctacagc    1380
accacagctg gccggcagat agcgagcggt ccggtggcga ccggcggcag cattactgtg    1440
ctggcccctg attcgtgtgt gccgtgccag ccacgggcga gcagtttctc atgcgggtca    1500
tcgcggagcg ttcggtttgc gtaa                                           1574

## Description

### TECHNICAL FIELD

The present invention relates to a keratin BD-10, a nucleic acid molecule encoding the keratin BD-10, an expression vector containing the nucleic acid molecule, a host cell containing the expression vector or the nucleic acid molecule integrated into genome, preparation method of the keratin BD-10, pharmaceutical composition containing this keratin, and the use of the keratin and the pharmaceutical composition in the preparation of medicament for antipyretic, analgesic, antitussive expectorant, anticonvulsant, antiepileptic, anti-hypertension, anti-inflammatory, and antiviral.

### BACKGROUND OF THE INVENTION

Keratin is a kind of protein, which is widely found in the epidermis of humans and animals, and is the main component of hair, feathers, hoofs, shells, claws, horns, etc. It is an extremely important structural protein for connective tissue and plays a role in protecting the body.

Keratin is widely present in organisms and is a renewable resource with great utilization value, but it has not been widely and effectively used. The main reason is that keratin is insoluble in various solvents, and keratin is generally more resistant to enzymatic hydrolysis by proteases than other proteins. Therefore, it is very difficult to extract and prepare natural keratin.

With the rapid development of modern biotechnology such as genomics, proteomics, genetic engineering, and microbial engineering, more and more genes have been discovered. The use of protein expression systems to prepare and produce target proteins is an important method for studying the biological functions of genes or proteins.

The target keratin is prepared by the protein expression system, and then used to study its structure and function. It has not been reported in other literature and is novel and has an inventive step.

### SUMMARY OF THE INVENTION

The technical problems solved by the present invention are to provide a keratin BD-10, a nucleic acid molecule encoding the keratin BD-10, an expression vector containing the nucleic acid molecule, and a host cell containing the expression vector or the nucleic acid molecule integrated into genome, and a preparation method of the keratin BD-10, a pharmaceutical composition containing the keratin BD-10, and use of the above-mentioned keratin BD-10, nucleic acid molecule, expression vector, host cell, or pharmaceutical composition in the preparation of medicament for antipyretic, analgesic, antitussive, expectorant, anticonvulsant, antiepileptic, anti-hypertension, anti-inflammatory, and antiviral.

In order to solve the technical problems of the present invention, the present invention provides the following technical solutions:
The first aspect of the technical solution of the present invention is to provide a keratin BD-10, cwherein the amino acid sequence of the keratin BD-10 is:
   (1) the amino acid sequence set forth in SEQ ID NO: 1 in the sequence listing.
   (2) an amino acid sequence that basically maintains the same biological function as the keratin BD-10 formed by substitution, deletion or addition of 1-35 amino acids to the amino acid sequence set forth in SEQ ID NO: 1 in the sequence listing.

Further, the keratin BD-10 can have a conventional modification; or the keratin BD-10 is further linked with a tag for detection or purification.

Furthermore, the conventional modification includes acetylation, amidation, cyclization, glycosylation, phosphorylation, alkylation, biotinylation, fluorescent group modification, polyethylene glycol PEG modification, immobilization modification, sulfation, oxidation, methylation, deamination, formation of disulfide bond or breakage of disulfide bond; The tag includes His6, GST, EGFP, NMP, Nus, HA, IgG, FLAG, c-Myc, Profinity eXact_{∘}

The second aspect of the technical solution of the present invention provides a nucleic acid molecule encoding the keratin BD-10 of the first aspect.

Further, the nucleotide sequence of the nucleic acid molecule is:
(1) the nucleotide sequence set forth in SEQ ID NO:2 in the sequence listing.
(2) a nucleotide sequences obtained by sequence optimization based on the nucleotide sequence set forth in SEQ ID NO:2.
(3) a nucleotide sequences complementary to the nucleotide sequence in (1) or (2) above.

The third aspect of the technical solution of the present invention provides an expression vector, wherein the expression vector contains the nucleic acid molecule described in the second aspect.

Further, the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the expression vector is preferably a pET series vector; the expression vector is more preferably pET-28a(+).

The fourth aspect of the technical solution of the present invention provides a host cell, wherein the host cell contains the expression vector of the third aspect or the nucleic acid molecule of the second aspect integrated into the genome.

Further, the host cell includes bacteria, yeast, aspergillus, plant cells, or insect cells. Furthermore, the bacteria include *Escherichia coli* or yeast.

Competent host cells can be BL21 series, Transetta series, Rosetta series, DH5α series, JM series, Top series, Orgami series, Trans1-T1, TG1, TB1; Y11430, MG1003, GS115 (AOX1), KM71, SMD1168, etc.; the preferred expression competent cells are BL21 (DE3), Transetta (DE3).

The fifth aspect of the technical solution of the present invention provides a method for preparing the keratin BD-10 of the first aspect, wherein the method comprises the following steps:
A. synthesizing a nucleic acid molecule corresponding to the keratin BD-10 described in the first aspect, linking the nucleic acid molecule to the corresponding expression vector, and transforming the expression vector into a host cell, culturing the host cell with the expression vector in a fermentation device under certain conditions and inducing the expression of the keratin BD-10 to obtain a crude protein solution containing the keratin BD-10;
B. subjecting the crude protein solution obtained in step A to separation and purification, and drying to obtain the keratin BD-10.

Further, in step A, the host cell is mainly selected from *Escherichia coli,* the keratin BD-10 is expressed in the inclusion bodies of *Escherichia coli,* and the fermentation device includes shake flask or fermenter.

Further, in step A, after inducing the expression of the keratin BD-10, impurities in which can be removed with a cleaning agent to obtain the crude protein solution by dissolving in a solution.

Further, the medium in step A may be LB medium, TB medium, SB medium, SOB medium, SOC medium, PDA medium, YPD medium, red bengal medium, high salt Chashi medium , DOBA medium, rice koji medium, and modified formula thereof, etc.; shake flask fermentation preferably LB medium, TB medium, most preferably TB medium; fermenter preferably LB medium and modified formula thereof.

Further, the inducer in step A can be IPTG, lactose, arabinose, etc.; preferably is IPTG or lactose.

Further, in step A, the obtained fermentation broth is centrifuged and then the supernatant is discarded; the precipitate is suspended in a buffer, the bacteria are broken, centrifuged again, and the supernatant is discarded; after the precipitate is washed with a cleaning agent, which is then dissolved in a urea solution to obtain the crude protein solution of BD-10.
Among them, the buffer is preferably buffer A, and its dosage is: fermentation broth volume : □ buffer A volume=1~100:1, preferably 10:1;
The cleaning agent can be urea solution, guanidine hydrochloride solution, triton and buffer A, etc., preferably urea solution, most preferably 2M urea solution (may contain 1% Triton). The dosage is: fermentation broth volume: 2M urea volume = 0.2~100:1, preferably 1~15:1;
The urea solution is preferably the 8M urea solution, and its dosage is: fermentation broth volume: 8M urea volume=0.2~100: 1, preferably 2~15: 1.

Further, in step B, the separation and purification method includes ultrafiltration microfiltration membrane technology purification method, column chromatography purification method, salting out method, and dialysis method.

Further, in step B, the separation and purification method is as follows:
(1)The dialysis method is to purify the crude protein solution obtained in step A by a dialysis method to obtain the target protein BD-10 solution.

The molecular weight cut-off of the dialysis bag can be 0.5-10 kD, the preferred molecular weight cut-off of the dialysis bag is 3.5-10 kD, and the most preferred molecular weight cut-off of the dialysis bag is 10 kD.

(2) The ultrafiltration and microfiltration method is to purify the crude protein solution obtained in step A by membrane technology such as an ultrafiltration membrane or a microfiltration membrane to obtain a concentrated solution of the target protein BD-10.

Preferably, the microfiltration membrane purification is performed twice, with the membrane pore size is 1000~1500 nm in the first time, and the membrane pore size is 20~50 nm in the second time.

(3) The column chromatography method is to pass the crude protein solution obtained in step A through a column chromatography, such as various exchange columns or exclusion column chromatography, to separate and purify the target protein BD-10.

The preferred exclusion column is dextran gel column, Superdex 30 Increase, Superdex 75 Increase, Superdex 200 Increase and Superose 6 Increase, etc.; the preferred exchange column is an ion exchange resin column: anion exchange resin column: HiTrap Q FF, HiTrap Capto Q ImpRes, Capto Q ImpRes, HiTrap Capto Q, HiTrap DEAE, Toyopearl Q-650M and Toyopearl SuperQ-650M, etc.; Cation exchange resin column: HiTrap SP FF, HiTrap Capto SP ImpRes, Capto SP ImpRes, HiTrap Capto SP, Toyopearl SP-650M and Toyopearl Super SP-650M. The most preferred is an anion exchange resin column.

As the eluent, commonly used eluents in the art can be used, such as water and salt solution. The salt solution includes sodium chloride solution, sodium dihydrogen phosphate solution, disodium hydrogen phosphate solution, sodium acetate, acetic acid, and the like.

(4)The salting-out method is to purify the crude protein solution obtained in step A by salting-out method to obtain the target protein BD-10 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of salting out is 1 to 3 times, preferably 2 times.

After salting out, the precipitate is washed with pure water, and the washing frequency is 2 to 5 times, preferably 3 times.

Further, the target protein BD-10 solution purified in step B can be freeze-dried or vacuum-dried into a dry powder, or the concentrated solution can be directly spray-dried into a dry powder.

The sixth aspect of the technical solution of the present invention provides a pharmaceutical composition, wherein the pharmaceutical composition contains the keratin BD-10 described in the first aspect or the nucleic acid molecule described in the second aspect or the expression vector of the third aspect or the host cell of the fourth aspect and a pharmaceutically acceptable carrier or excipient.

The keratin obtained in the above steps of the present invention can be freeze-dried or vacuum-dried into a dry powder, or the concentrated liquid can be directly spray-dried into a dry powder, and then made into various dosage forms.

The present invention relates to a pharmaceutical composition, which comprises any keratin obtained in the above steps and a pharmaceutically acceptable carrier.

The present invention also relates to a pharmaceutical composition containing the keratin of the present invention as an active ingredient and conventional pharmaceutical excipients or adjuvants. Generally, the keratin of the present invention accounts for 0.1-100.0% of the total weight of the pharmaceutical composition.

The present invention also provides a pharmaceutical composition, which includes a pharmaceutical effective dose of protein as an active ingredient and a pharmaceutically acceptable carrier.

The pharmaceutical composition of the present invention can be prepared according to methods recognized in the field. When used for this purpose, if necessary, the protein of the present invention can be combined with one or more solid or liquid pharmaceutical excipients and/or adjuvants to prepare an appropriate administration form or dosage that can be used as human or veterinary drugs form.

The keratin of the present invention or the pharmaceutical composition containing the same can be administered in a unit dosage form. The route of administration can be enteral or parenteral, such as oral administration, intramuscular, subcutaneous, nasal cavity, oral mucosa, eye, lung, skin, vagina, peritoneum and rectum, etc., oral administration is preferred.

The keratin protein of the present invention or the pharmaceutical composition containing the same can be administered by injection. Injection includes intravenous injection, intramuscular injection, subcutaneous injection, intradermal injection, intraperitoneal injection, and acupoint injection, etc.

The dosage form for administration may be a liquid dosage form, a solid dosage form or a semi-solid dosage form. Liquid dosage form can be solution (including true solution and colloidal solution), emulsion (including oil-in-water, water-in-oil and double emulsion), suspension, injection (including water injection, powder injection and infusion), eye drops Lotion, nasal drops, lotion and liniment, etc. The solid dosage form can be tablet (including ordinary tablet, enteric-coated tablet, buccal tablet, dispersible tablet, chewable tablet, effervescent tablet, orally disintegrating tablet), capsule (including hard capsule, soft capsule, and enteric-coated capsule), granules preparation, powder, pellet, dripping pill, suppositorie, film, patche, air (powder) spray, spray, etc.; semi-solid dosage form can be ointment, gel, paste, etc.

The keratin of the present invention can be made into ordinary preparations, slow-release preparations, controlled-release preparations, targeted preparations, and various particle delivery systems.

In order to make a unit dosage form into a tablet, various excipients known in the art can be widely used, including diluents, binders, wetting agents, disintegrants, lubricants and glidants. The diluent can be starch, dextrin, sucrose, glucose, lactose, mannitol, sorbitol, xylitol, microcrystalline cellulose, calcium sulfate, calcium hydrogen phosphate, calcium carbonate, etc.; the humectant can be water, ethanol, iso Propanol, etc.; the binder can be starch syrup, dextrin, syrup, honey, glucose solution, microcrystalline cellulose, acacia syrup, gelatin syrup, sodium carboxymethyl cellulose, methyl cellulose, hypromellose Base cellulose, ethyl cellulose, acrylic resin, carbomer, polyvinylpyrrolidone, polyethylene dipropanol, etc.; disintegrant can be dry starch, microcrystalline cellulose, low-substituted hydroxypropyl cellulose , Cross-linked polyvinylpyrrolidone, cross-linked sodium carboxymethyl cellulose, sodium carboxymethyl starch, sodium bicarbonate and rafter acid, calcium carbonate, polyoxyethylene sorbitol fatty acid ester, dodecyl Sodium sulfonate; lubricant and glidant can be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multi-layer tablets.

In order to make the administration unit into a pill, various carriers known in the field can be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methyl cellulose, ethyl cellulose and so on.

In order to make the administration unit into a suppository, various carriers known in the field can be widely used. Examples of carriers are, for example, polyethylene glycol, lecithin, cocoa butter, higher alcohols, higher alcohol esters, gelatin, semi-synthetic glycerides and the like.

In order to make the dosing unit into a capsule, the active ingredient keratin of the present invention is mixed with the above-mentioned various carriers, and the resulting mixture is placed in hard gelatin capsules or soft capsules. The active ingredient keratin of the present invention can also be made into microcapsules, suspended in an aqueous medium to form a suspension, or filled into hard capsules or made into injections for application.

For example, the keratin of the present invention is prepared into injection preparations, such as solutions, suspension solutions, emulsions and freeze-dried powder injections. Such preparations may be aqueous or non-aqueous, and may contain one and/or more pharmacodynamically acceptable carriers, diluents, binders, lubricants, preservatives, surfactants or dispersants. For example, the diluent can be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin can be added to the injection preparation. In addition, conventional solubilizers, buffers, pH adjusters, etc. can also be added. These auxiliary materials are commonly used in this field.

In addition, if necessary, coloring agents, preservatives, flavors, flavors, sweeteners or other materials can also be added to the pharmaceutical preparations.

In order to achieve the purpose of medication and enhance the therapeutic effect, the keratin or the pharmaceutical composition of the present invention can be administered by any known administration method.

The dosage of the keratin pharmaceutical composition of the present invention depends on many factors, such as the nature and severity of the disease to be prevented or treated, the gender, age, weight, personality and individual response of the patient or animal, the route of administration, the number of administrations and the purpose of treatment, so the therapeutic dose of the present invention can have a wide range of changes. Generally speaking, the dosage of the pharmaceutical ingredients of the present invention is well known to those skilled in the art. Appropriate adjustments can be made according to the actual amount of the drug contained in the final preparation in the keratin composition of the present invention to meet the requirement of the therapeutically effective amount and accomplish the preventive or therapeutic purpose of the present invention. The appropriate daily dosage range of keratin of the present invention: the dosage of keratin of the present invention is 0.01~500 mg/kg body weight, preferably 0.5~100 mg/kg body weight, more preferably 1~50 mg/kg body weight, and most preferably 2~30 mg/kg body weight. The above dosage can be administered in a single dosage form or divided into several, such as two, three or four dosage forms, depending on the clinical experience of the administering doctor and the dosage regimens including the use of other treatments. The total dose required for each treatment can be divided into multiple or single doses. The protein or pharmaceutical composition of the present invention can be taken alone, or combined with other therapeutic drugs or symptomatic drugs and the dosage can be adjusted.

The seventh aspect of the technical solution of the present invention provides the use of the keratin BD-10 of the first aspect or the nucleic acid molecule of the second aspect or the expression vector of the third aspect or the host cell of the fourth aspect or the pharmaceutical composition of the sixth aspect in the preparation of medicament for antipyretic, analgesic, antitussive, expectorant, anticonvulsant, antiepileptic, anti-hypertension, anti-inflammatory, and antiviral.

In order to accomplish the purpose of the present invention, the present invention takes the following technical solutions. Specifically, the preparation method of the keratin BD-10 of the present invention includes the following steps:
(1) Synthesizing the nucleotide sequence and determining the accuracy of the sequence;
   The preferred nucleotide sequence is set forth in SEQ ID NO:2.
(2) Transferring the nucleotide sequence into an expression vector;
   The expression vector can be pET series, pUC series, pQE series, pBV series, pMAL series, pPIC9K, pHIL-S1, pPICZα/A, pYAM75P, pHIL-D2, pA0815, pPIC3K, pPICZ, pHWO10, pGAPZ, pGAPZa, pPIC3.5K ect. The preferred expression vector is a pET series vector; the most preferred expression vector is pET-28a(+).
(3) Transfecting the expression vector into a host cell;
   The host cell can be *E. coli* or yeast; the preferred host cell is *E. coli;*
   Competent cells can be BL21 series, Transetta series, Rosetta series, DH5α series, JM series, Top series, Orgami series, Trans1-T1, TGI, TB1; Y11430, MG1003, GS115 (AOX1), KM71, SMD1168, ect. Preferred expression competent cell is BL21 (DE3) or transetta (DE3).
(4) Culturing the host cell under appropriate conditions to induce the expression of the target protein BD-10;
   Fermentation device can use shake flask or fermenter;
   The medium can be LB medium, TB medium, SB medium, SOB medium, SOC medium, PDA medium, YPD medium, red bengal medium, high salt Chashi medium, DOBA medium, rice koji culture medium, and their improved formulas, etc.; shake flask fermentation preferably LB medium, TB medium, and most preferably TB medium; fermenter preferably LB medium and its improved formulas.
   The inducer can be IPTG, lactose, arabinose, etc.; preferably is IPTG or lactose.
(5) Enriching the target protein BD-10 product;
   Centrifuge the fermented bacterial liquid obtained in step (4), and discard the supernatant; suspend the precipitate in the buffer, then crush the bacteria, and then centrifuge again, and discard the supernatant; after washing the precipitate with a cleaning agent, dissolved with urea solution to obtain BD-10 crude protein solution.
   Among them, the buffer is preferably buffer A, and its dosage is: fermentation broth volume : buffer A volume=1~100 : 1, preferably 10: 1;
   The cleaning agent can be urea solution, guanidine hydrochloride solution, Triton, buffer A, etc., preferably urea solution, most preferably 2M urea solution (may contain 1% Triton). The dosage is fermentation broth volume: 2M urea volume=0.2~100: 1, preferably 1~15: 1;
   The urea solution is preferably an 8M urea solution. Its dosage is the volume of the fermentation broth: 8M urea volume=0.2~100:1, preferably 2~15: 1.
(6) Separation and purification of the target protein BD-10.
   The crude protein solution obtained in step (5) needs to be purified to obtain the target protein BD-10. The purification can be carried out by dialysis, or ultrafiltration and microfiltration, or column chromatography, or salting out steps.

A. In the dialysis step, the crude protein solution obtained in step (5) is purified by a dialysis method to obtain the target protein BD-10 solution.

The molecular weight cut-off of the dialysis bag can be 0.5-10 kD, the preferred molecular weight cut-off of the dialysis bag is 3.5-10 kD, and the most preferred molecular weight cut-off of the dialysis bag is 10 kD.

B. In the ultrafiltration and microfiltration step, the crude protein solution obtained in step (5) is purified by membrane technology such as ultrafiltration membrane or microfiltration membrane to obtain the concentrated solution of the target protein BD-10.

Preferably, the microfiltration membrane purification is performed twice, with the membrane pore size is 1000~1500nm in the first time, and the membrane pore size is 20~50nm in the second time.

C. In the column chromatography step, the crude protein solution obtained in step (5) is passed through column chromatography, such as various exchange columns or exclusion column chromatography, to separate and purify the target protein BD-10.

The preferred exclusion column is dextran gel column, Superdex 30 Increase, Superdex 75 Increase, Superdex 200 Increase, Superose 6 Increase, etc.; The preferred exchange column is an ion exchange resin column: anion exchange resin column, HiTrap Q FF, HiTrap Capto Q ImpRes, Capto Q ImpRes, HiTrap Capto Q, HiTrap DEAE, Toyopearl Q-650M, Toyopearl SuperQ-650M, etc.; Cation exchange resin column, HiTrap SP FF, HiTrap Capto SP ImpRes, Capto SP ImpRes, HiTrap Capto SP, Toyopearl SP-650M, Toyopearl Super SP-650M. The most preferred is an anion exchange resin column.

As the eluent, commonly used eluents in the art can be used, such as water, salt solution, and the salt solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein BD-10 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of salting out is 1 to 3 times, preferably 2 times.

After salting out, the precipitate is washed with pure water, and the washing frequency is 2 to 5 times, preferably 3 times.

The target protein BD-10 solution purified from steps A to D can be freeze-dried or vacuum dried into dry powder, or the concentrated solution can be directly spray-dried into dry powder.

The beneficial technical effects of the present invention:
1. The protein of the present invention is the keratin obtained for the first time, and the preparation method of the present invention has the characteristics of high yield and high sample purity.
2. The pharmacodynamic test study of protein BD-10 on lipopolysaccharide (LPS) induced fever model in SD rats proves that protein BD-10 has a significant effect of reducing body temperature rise 2 hours after modeling; Through the pharmacodynamic study of protein BD-10 on the yeast-induced fever model of SD rats, it is proved that protein BD-10 can significantly inhibit the increase in body temperature at 2 hours, 4 hours, 6 hours, and 8 hours after modeling, and has a strong effect;
3. In the present invention, the pharmacodynamic test study of protein BD-10 on pilocarpine (PLO) induced convulsions and epilepsy in mice respectively, proved that protein BD-10 can significantly prolong the incubation period of Grade III epilepsy in mice;
4. The present invention proves that the protein BD-10 has obvious expectorant effect through the experimental study of the pharmacological effect of protein BD-10 on the expectorant of phenol red excretion method in mice;
5. In the present invention, the pharmacodynamic study of protein BD-10 on the antitussive effect of the method of inducing cough with ammonia water in mice prove that the protein BD-10 has the tendency to reduce the number of coughs and prolong the incubation period, and has the potential of antitussive effect;
6. The present invention proves that the protein BD-10 can significantly reduce the number of writhing times in mice and has a significant analgesic effect through the pharmacodynamic test study of the protein BD-10 on the acetic acid writhing of ICR mice.

### DRAWINGS

Figure 1: Analysis of reduced SDS polyacrylamide gel electrophoresis (SDS-PAGE) of expressed protein BD-10.
   (M: Protein molecular weight standard; S: Expressed protein BD-10)
Figure 2: Effect of protein BD-10 on lipopolysaccharide (LPS) induced fever in rats.
   (Compared with normal control group, ^{∗∗∗} P<0.001; Compared with the model group, ## P<0.01, ###P<0.001)
Figure 3: Effect of protein BD-10 on yeast-induced fever model in rats.
   (Compared with normal control group, ^{∗∗} P<0.01, ^{∗∗∗} P<0.001; Compared with the model group, # P<0.05, ## P<0.01, ### P<0.001).

### DETAILED EMBODIMENTS

The following examples and pharmacological activity test examples are used to further illustrate the present invention, but this does not mean any limitation to the present invention.

The experimental methods in the following examples and pharmacological activity test examples are conventional methods unless otherwise specified; the experimental materials used, unless otherwise specified, are purchased from conventional biochemical reagent companies.

### Example 1 Shake flask fermentation to prepare protein BD-10 crude solution A (TB medium)

Synthesize the nucleotide sequence set forth in SEQ ID NO:2 and transfer it into the pET-28a(+) vector; confirm the sequence to obtain an expression vector containing the correct sequence; transfect the expression vector into BL21 (DE3) cells, obtain expression competent host cells containing the target nucleotide sequence. Add LB medium and incubate in a shaker at 37°C and 220 rpm for 1 hour to obtain a recombinant strain.

Dip the recombinant strain and streak it on an LBA plate containing Kanamycin, and place the plate upside down in a 37°C constant temperature incubator overnight for 16 hours.

Configure 400 ml of TB medium, divided into 2 bottles, each bottle of 200 ml. Add Kanamycin (final concentration 50 µg/ml) to each bottle (200 ml) of TB medium. Take a single colony on the plate and add it to the TB medium. Amplify and culture overnight at 37°C and 220 rpm to obtain seed liquid in the shaker.

Configure 28.8 L TB medium, divided into 144 bottles, each bottle of 200 ml. Add Kanamycin (final concentration 50 µg/ml) to each bottle (200 ml) of TB medium, then add 2 ml of seed solution, and incubate in a shaker at 37°C and 220 rpm for 2-3 hours. Monitor the OD₆₀₀, when the OD₆₀₀ reaches about 1.0, add an inducer to induce protein expression in the shaker, and the induction conditions are selected from the following table.

| | Inducer | Induction temperature | Induction time | Shaker speed |
|---|---|---|---|---|
| Induction conditions | IPTG(Final concentration 0.5mM) | 16°C | 16 h | 220 rpm |
| | | 25°C | 8 h | |
| | | 37°C | 5 h | |

Combine each bottle of bacterial liquid, centrifuge at 7000 rpm for 5 minutes, and discard the supernatant after sterilization; the precipitate is suspended in about 3 L of buffer, filtered with an 80-100 mesh screen, and the filtrate is crushed with a high-pressure crusher at a pressure of 800-1000 bar, twice, 2 minutes each time. Centrifuge the broken bacteria liquid at 7000 rpm for 30 minutes, discard the supernatant, and obtain the precipitate (ie inclusion body). The precipitate was washed twice with 1L detergent, centrifuged and the supernatant was discarded. The precipitate was dissolved in urea solution 4 times, respectively, 800 ml, 600 ml, 400 ml and 400 ml. The four solutions were combined and centrifuged at 7000 rpm for 30 minutes. The precipitate was discarded and the supernatant was the crude protein solution A.

| Buffer | cleaning agent | Urea solution |
|---|---|---|
| buffer A | 2M urea solution (may contain Triton) | 8M urea solution (may contain Tris/HCl buffer or NaH₂PO₄/Na₂HPO₄ Buffer) |
| | 4M urea solution (may contain Triton) | |
| | 2M Guanidine Hydrochloride Solution | |
| | 4M Guanidine Hydrochloride Solution | 4M urea solution (may contain Tris/HCl buffer) |

Protein BD-10 crude solution A was analyzed by reduced SDS-PAGE. The separation gel concentration was 12.5% and then stained with Coomassie brilliant blue R250 method; a clear blue band is shown near the molecular weight of 55 kD.

### Example 2 Shake flask fermentation to prepare protein BD-10 crude solution B (other medium)

In Example 1, it was synthesized and sequenced to confirm that an expression vector containing the sequence set forth in SEQ ID NO:2 was obtained; the expression vector was transfected into Transetta (DE3) cells to obtain expression-competent host cells containing the target nucleotide sequence.

Prepare 20 ml of LB medium, take 800 µl, add 50 µl of host cells containing the target coding sequence, and incubate at 37° C and 220 rpm for 1 hour in a shaker.

Dip the above bacterial liquid and streak it on an LBA plate containing Kanamycin, and place the plate upside down in a 37°C constant temperature incubator overnight for 16 hours.

Take 10 ml of LB medium, add Kanamycin (final concentration 50 µg/ml), take a single colony on the plate and add it to the LB medium. Amplify and culture overnight at 37°C and 220 rpm for 15 hours to obtain seed liquid in a shaker.

Configure 1 L of the medium shown in the table below, and divide it into 10 bottles of 100 ml each. Add Kanamycin (final concentration 50 µg/ml) to each bottle (100 ml) of medium and then add 1 ml of seed solution. Incubate at 37°C and 220 rpm for 2-3 hours in a shaker. Monitor OD₆₀₀ and add inducer IPTG (final concentration 0.5 mM) when OD₆₀₀ reaches about 1.0. Induce protein expression at 37°C and 220 rpm in a shaker.

| | |
|---|---|
| Culture medium | LB medium, SOB medium, SOC medium |

Combine each bottle of bacterial liquid, centrifuge at 10000 rpm for 10 minutes, and discard the supernatant after sterilization; the precipitate is suspended in about 100 mL of buffer, filtered with an 80-100 mesh screen, and the filtrate is crushed with a high-pressure crusher at a pressure of 800-1000 bar, twice, 2 minutes each time. Centrifuge the broken bacteria liquid at 10000 rpm for 30 minutes and discard the supernatant.

Add 40 mL of cleaning agent buffer A to the precipitate for washing 3 times, centrifuge and discard the supernatant; Add 40 mL of cleaning agent 2M urea solution to the precipitate to wash twice, centrifuge, and discard the supernatant; the precipitate is then added to 8M urea solution (containing 50mM Tris/HCl buffer) to dissolve 3 times, respectively, 40 ml, 30 ml, 30 ml; the combined solutions were centrifuged at 7000 rpm for 30 minutes, the precipitate was discarded, and the supernatant was the crude protein solution B.

Protein BD-10 crude solution B was analyzed by reduced SDS-PAGE wtih the separation gel concentration was 12.5% and then stained with Coomassie brilliant blue R250 method; a clear blue band is shown near the molecular weight of 55 kD.

### Example 3 Preparation of crude protein BD-10 solution C in a fermenter

In Example 1, it was synthesized and sequenced to confirm that an expression vector containing the sequence set forth in SEQ ID NO:2 was obtained; the expression vector was transfected into BL21 (DE3) cells to obtain expression-competent host cells containing the target nucleotide sequence. Add the expression-competent host cells in LB medium and incubate in a shaker at 37°C and 220 rpm for 1 hour to obtain a recombinant strain.

In the LBA plate containing Kanamycin, add 100 µl of the recombinant strain, spread with spreader until it becomes evenly dry, and place the plate upside down in a constant temperature incubator at 37°C for overnight culture. Take three single colonies, streak them on a plate containing Kanamycin, and then culture the plate overnight. After three batches of shake flask fermentation and expression verification are confirmed to be correct, the strains are preserved with 15% glycerol and divided into 0.8 ml each to obtain a working cell bank, which is stored in a refrigerator at -80°C for later use.

Take out 1 glycerol bacteria from the working cell bank, take 100 µl, and add it to 40 ml LB medium, add Kanamycin (final concentration 50 µg/ml), incubate in a shaker at 37°C and 220 rpm for 6 hours to obtain a first-level seed solution.

Take 1.2ml of the first-level seed solution, add it to 120 ml LB medium, add Kanamycin (final concentration 50 µg/ml), and then incubate in a shaker at 37°C and 220 rpm for 6 hours to obtain a second-level seed solution.

Add 3L of modified LB broth to a 5L fermenter, then add 120 ml of the second-level seed solution, 3 ml of Kanamycin (final concentration 50 µg/ml), and incubateat 37°C and 30% dissolved oxygen (series speed) for about 8 hours. Monitor the OD value around 20 and 3g lactose as an inducer. Induction was performed at 20°C, fed at a rate of 30 ml/hour, and incubated at 20°C for 24 hours.

Centrifuge the bacterial solution at 7000 rpm for 5 minutes, and discard the supernatant after sterilization; the precipitate is suspended in about 200 mL of buffer A, filtered with an 80-100 mesh screen, and the filtrate is crushed with a high-pressure crusher at a pressure of 800-1000 bar, twice, 2 minutes each time. Centrifuge the broken bacteria liquid at 7000 rpm for 30 minutes and discard the supernatant.

Add 2M urea solution (including 1% Triton) to the precipitate and wash it twice, 1L each time; then add 1L 2M urea solution to wash once, centrifuge and discard the supernatant. The precipitate is then added to 8M urea solution (containing 50mM Tris/HCl buffer) to dissolve 4 times, respectively, 400 ml, 300 ml, 200 ml, 100 ml; the four solutions were combined, centrifuged at 7000 rpm for 30 minutes, the precipitate was discarded, and the supernatant was the crude protein solution C.

Protein BD-10 crude solution C was analyzed by reduced SDS-PAGE with the separation gel concentration was 12.5% and then stained with Coomassie brilliant blue R250 method; a clear blue band is shown near the molecular weight of 55 kD.

### Example 4 Protein BD-10 was prepared from crude protein solution A by dialysis.

The crude protein solution A obtained in Example 1 was filtered with a 0.55 µm filter membrane, and the filtrate was combined. The filtrate was dialyzed with water, the molecular weight cut-off of the dialysis bag was 10kD, dialyzed for 72 hours, and the inner liquid was freeze-dried to obtain the target protein BD-10; the purity measured by electrophoresis was 96.8%.

### Confirmation of protein BD-10 structure:

1 Reduced SDS-polyacrylamide gel electrophoresis (SDS-PAGE) analysis Instrument: Protein electrophoresis (Bio-Rad).
   Methods and results: The protein BD-10 solution was analyzed by reduced SDS-PAGE, the separation gel concentration was 12.5%, and it was stained with Coomassie brilliant blue R250 method. The molecular weight of BD-10 band is around 55 kD.
2 Complete protein sequence analysis based on LC-MS/MS
   Main materials: Acetonitrile, formic acid, ammonium bicarbonate, dithiothreitol (DTT), iodoacetamide (IAA), trypsin, chymotrypsin, Glu-C, Asp-N;
   Main instruments: Capillary High Performance Liquid Chromatograph (Thermo Ultimate 3000), Electrospray-Combined Ion Trap Orbitrap Mass Spectrometer (Thermo Q Exative Hybrid Quadrupole-Orbitrap Mass Spectrometer).
   Methods and results:
      Protein BD-10 undergoes pre-treatments such as dissolution replacement, reductive alkylation, and various proteolysis to obtain enzyme-cleaved peptides; Restriction digestion peptide solution was analyzed by liquid chromatography tandem mass spectrometry. The original mass spectrometry file uses Maxquant (1.6.2.10) to search the protein database to analyze the data. The identification result was determined to be consistent with the target sequence SEQ ID NO:1.

### Example 5 Protein crude solution A is purified by other methods to prepare protein BD-10.

The crude protein solution A obtained in Example 1 was purified by the following two methods:
The first method: salting out;

The crude protein solution A is placed in a stirred container for two salting out: Slowly add saturated ammonium sulfate solution along the wall to make the final concentration of ammonium sulfate 25% or 50%. During the salting-out process, the protein is separated out. After the salting-out is complete, filter to complete the first salting-out; Add 400 ml of pure water to the precipitate to suspend, and then slowly add a saturated solution of ammonium sulfate along the wall to make the final concentration of ammonium sulfate 25%. Carry out the second salting out, filtration, and the precipitate is the crude protein extract. The crude protein extract was washed three times with water: add 200 ml of pure water to suspend, stir, let stand, and filter; After this is repeated three times, the precipitate is freeze-dried to obtain the target protein BD-10.
The second method: column chromatography;

The crude protein solution A is purified by anion exchange resin column, such as HiTrap Q FF 16/10, HiTrap Capto Q ImpRes, Capto Q ImpRes, HiTrap Capto Q, HiTrap DEAE, etc. The eluent is a gradient elution of NaCl solution, plus 20mM NaH₂PO₄/Na₂]HPO₄ buffer (pH 8.0). The elution fractions are combined according to the results of SDS-PAGE electrophoresis detection. The combined eluate was centrifuged twice at 7000 rpm for 1 hour each time; The supernatant was filtered with a 0.45 µm filter membrane, and the filtrates were combined. The filtrates are concentrated by dialysis with water, the molecular weight cut-off of the dialysis bag is 10 kD, and the inner liquid is freeze-dried to obtain the target protein BD-10.

The product protein BD-10 obtained by the two methods was confirmed to have the same amino acid sequence as the protein prepared in Example 4 through the same structural confirmation method as in Example 4.

### Example 6 Protein BD-10 was prepared by purified protein crude solution B

The crude protein solution B obtained in Example 2 was purified by the following three methods:
The first method: dialysis;

The crude protein solution B is filtered with a 0.45 µm membrane, the filtrate is dialyzed with water, dialyzed for more than 72 hours, and the inner solution is freeze-dried to obtain the target protein BD-10.

| | |
|---|---|
| Dialysis bag | Molecular weight cut-off: 0.5 kD, 3.5 kD, 5 kD, 10 kD |

The second method: column chromatography;

The crude protein solution B is purified by anion exchange resin column, such as HiTrap Q FF 16/10, HiTrap Capto Q ImpRes, Capto Q ImpRes, HiTrap Capto Q, HiTrap DEAE, etc. The eluent is a gradient elution of NaCl solution, plus 20mM NaH₂PO₄/Na₂HPO₄ buffer (pH 8.0). The elution fractions are combined according to the results of SDS-PAGE electrophoresis detection. The combined eluate was centrifuged twice at 7000 rpm for 1 hour each time; The supernatant was filtered with a 0.45 µm filter membrane, and the filtrates were combined. The filtrates are concentrated by dialysis with water, the molecular weight cut-off of the dialysis bag is 10 kD, and the inner liquid is freeze-dried to obtain the target protein BD-10.
The third method: salting out;

The crude protein solution B is placed in a stirred container for two salting out: Slowly add saturated ammonium sulfate solution along the wall to make the final concentration of ammonium sulfate 25% or 50%. During the salting-out process, the protein is separated out. After the salting-out is complete, filter to complete the first salting-out; Add 400 ml of pure water to the precipitate to suspend, and then slowly add a saturated solution of ammonium sulfate along the wall to make the final concentration of ammonium sulfate 25%. Carry out the second salting out, filtration, and the precipitate is the crude protein extract. The crude protein extract was washed three times with water: add 200 ml of pure water to suspend, stir, let stand, and filter; After this is repeated three times, the precipitate is freeze-dried to obtain the target protein BD-10.

The product protein BD-10 obtained by the three methods was confirmed to have the same amino acid sequence as the protein prepared in Example 4 through the same structural confirmation method as in Example 4.

### Example 7 Protein BD-10 was prepared by purification of crude protein solution C

The crude protein solution C obtained in Example 3 was purified by the following two methods:
The first method: Microfiltration membrane technology;

The crude protein solution C is purified by microfiltration membrane technology: first use a 1500 nm or 1000 nm ceramic membrane core for solid-liquid separation; discard the inner liquid, and then use a 20 nm or 50 nm ceramic membrane core for repeated microfiltration to remove urea; The inner liquid of the second microfiltration is freeze-dried to obtain the target protein BD-10.

The second method: salting out;
The crude protein solution C was placed in a container with stirring for two salting out: Slowly add saturated ammonium sulfate solution along the wall to make the final concentration of ammonium sulfate 25%. During the salting-out process, the protein is separated out. After the salting-out is complete, filter to complete the first salting-out; Add 400 ml of pure water to the precipitate to suspend, and then slowly add a saturated solution of ammonium sulfate along the wall to make the final concentration of ammonium sulfate 25%. Carry out the second salting out, filtration, and the precipitate is the crude protein extract. The crude protein extract was washed three times with water: add 200 ml of pure water to suspend, stir, let stand, and filter; After this is repeated three times, the precipitate is freeze-dried to obtain the target protein BD-10.

The product protein BD-10 obtained by the two methods was confirmed to have the same amino acid sequence as the protein prepared in Example 4 through the same structural confirmation method as in Example 4.

### Pharmacological test

### Experimental example 1 The pharmacodynamic test of protein BD-10 (Example 4 protein) on lipopolysaccharide (LPS) induced fever in SD rats.

Animals: 230-260 grams of male SD rats;
Drugs: lipopolysaccharide (LPS, SIGMAL-2880), aspirin (SIGMA A2093), protein BD-10; Instruments: electronic balance (SARTORIUS BP121S type), electronic clinical thermometer (CITIZEN CT-513W type).
Experiment grouping:
   Normal control group;
   Model group: lipopolysaccharide fever model;
   Positive control group: Aspirin 300 mg/kg group;
   Protein BD-10, 10 mg/kg group, 50 mg/kg group.
Method: the method of intraperitoneal injection of Lipopolysaccharide to replicate rat fever model.

Preparation of experimental animals: After the experimental animals adapt to the experimental environment (temperature 22°C±2°C, relative humidity 50%±2%) for 1 day. Pre-adaptation to measure rectal temperature at 8:00 and 15:00, rats were fasted and water was taken freely 12h before experiment, and let the animal to empty its feces before measuring the rectal temperature. Apply petroleum jelly to the electronic thermometer probe before each temperature measurement. Insert the rat rectum 2 cm (can be marked at 2 cm to ensure that the depth of each insertion is consistent), and record the body temperature after the reading is stable.

Intraperitoneal injection of lipopolysaccharide to replicate rat fever model: The body temperature of the rats was measured before modeling. Qualified rats with a body temperature of 36.2-37.3°C were selected and randomly divided into groups with 8 rats in each group. After oral administration of aspirin and different doses of protein BD-10, lipopolysaccharide (20 µg/kg, 2 ml/kg) was injected intraperitoneally immediately, and the normal control group was injected intraperitoneally with an equal volume of normal saline. The body temperatures of the rats were monitored after 2 hours for a total of 8 hours.

Statistics:
According to the body temperature measured at each time point on the day of the experiment, calculate the mean, standard deviation and standard error of the body temperature of each group of rats. The data of each group was compared with TTEST, and P<0.05 was considered as a significant difference.

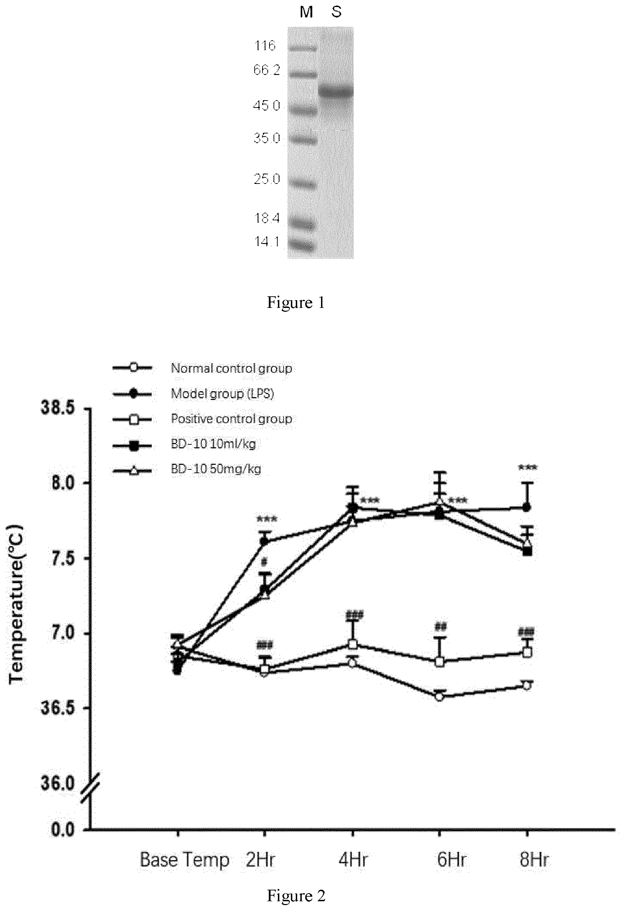

Experimental results:
Immediately after oral administration of aspirin (300 mg/kg), protein BD-10 (10 mg/kg, 50 mg/kg), intraperitoneal injection of 20 µg/kg lipopolysaccharide was performed to establish a model. The animal body temperature was monitored at 2 hours, 4 hours, 6 hours, and 8 hours after modeling. The results are shown in Table 1 and Figure 2.

**Table 1. Effects of test drugs on lipopolysaccharide (LPS) induced fever model in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.9±0.1 | 36.7±0.1 | 36.8±0.05 | 36.6±0.05 | 36.7±0.03 |
| Model group | 8 | 36.8±0.1 | 37.6±0.1^{∗∗∗} | 37.8±0.1^{∗∗∗} | 37.8±0.2^{∗∗∗} | 37.8±0.2^{∗∗∗} |
| Positive control group | 8 | 36.9±0.1 | 36.8±0.1### | 36.9±0.2### | 36.8±0.2## | 36.9±0.1 ### |
| BD-10-10 mg/kg | 8 | 36.8±0.1 | 37.3±0.1# | 37.8±0.1 | 37.8±0.1 | 37.6±0.1 |
| BD-10-50 mg/kg | 8 | 36.9±0.1 | 37.3±0.2 | 37.7±0.2 | 37.9±0.2 | 37.6±0.1 |

| | | | | | | |
|---|---|---|---|---|---|---|
| (Compared with the normal control group, ^{∗∗∗} P<0.001; compared with the model group, # P<0.05, ## P<0.01, ### P<0.001) | | | | | | |

Experimental results:
Immediately after oral administration of aspirin (300 mg/kg), protein BD-10 (10 mg/kg, 50 mg/kg), respectively, intraperitoneal injection of 20 µg/kg lipopolysaccharide was performed to establish a model. The animal body temperature was monitored at 2 hours, 4 hours, 6 hours, and 8 hours after modeling.. The results show that:
1) Intraperitoneal injection of 20 µg/kg lipopolysaccharide can successfully induce the increase of body temperature in rats. The body temperature of rats in the model group increased significantly at 2 hours, 4 hours, 6 hours, and 8 hours after modeling. Compared with the normal group, P<0.05, there is a statistical difference, and the model is stable.
2) The positive tool drug aspirin group can effectively inhibit the increase in body temperature of model rats at 2 hours, 4 hours, 6 hours, and 8 hours after modeling. Compared with the model group, P<0.05, there is a statistical difference, and the performance of positive tool drugs is relatively stable.
3) Protein BD-10 10mg/kg dose group can significantly reduce the body temperature of model rats 2 hours after modeling, and compared with the model group, P<0.05, there is a statistical difference.

### Experimental example 2 The pharmacodynamic test of protein BD-10 (the protein in Example 4) on the fever model of SD rats induced by yeast Animals: 230-260 grams of male SD rats;

Medicines: yeast (OXOID LP0021), aspirin (SIGMAA2093), protein BD-10;
Instruments: electronic balance (SARTORIUS BP121S type), electronic clinical thermometer (CITIZEN CT-513W type).
Experiment grouping:
   Normal control group;
   Model group: yeast fever model;
   Positive control group: Aspirin 300 mg/kg group;
   Protein BD-10, 10 mg/kg group, 50 mg/kg group.
Method:
   Preparation of experimental animals: After the experimental animals adapt to the experimental environment (temperature 22°C±2°C, relative humidity 50%±2%) for 1 day. Pre-adaptation to measure rectal temperature at 8:00 and 15:00, rats were fasted and water was taken freely 12h before experiment, and let the animal to empty its feces before measuring the rectal temperature. Apply petroleum jelly to the electronic thermometer probe before each temperature measurement. Insert the rat rectum 2 cm (can be marked at 2 cm to ensure that the depth of each insertion is consistent), and record the body temperature after the reading is stable.

Subcutaneous injection of dry yeast to replicate rat fever model: The body temperature of the rats was measured before modeling. Qualified rats with a body temperature of 36.2-37.3°C were selected and randomly divided into groups with 8 rats in each group. After oral administration of aspirin and different doses of protein BD-10, 20% yeast suspension (10 ml/kg) was injected subcutaneously immediately, and the normal control group was injected intraperitoneally with an equal volume of normal saline. The body temperatures of the rats were monitored after 2 hours for a total of 8 hours.

Statistics:
According to the body temperature measured at each time point on the day of the experiment, calculate the mean, standard deviation and standard error of the body temperature of each group of rats. The data of each group was compared with TTEST, and P<0.05 was considered as a significant difference.

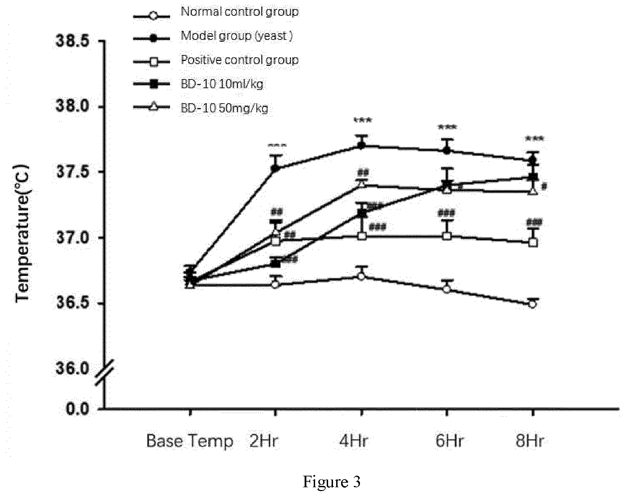

Experimental results:
Immediately after oral administration of aspirin (300 mg/kg), protein BD-10 (10 mg/kg, 50 mg/kg), subcutaneous injection of 20% yeast was performed to establish a model. The animal body temperature was monitored at 2 hours, 4 hours, 6 hours, and 8 hours after modeling. The results are shown in Table 2 and Figure 3.

**Table 2. Effects of the tested drugs on the yeast-induced fever model in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.6±0.1 | 36.6±0.1 | 36.7±0.1 | 36.6±0.1 | 36.5±0.04 |
| Model group | 8 | 36.7±0.1 | 37.5±0.1^{∗∗∗} | 37.7±0.1^{∗∗∗} | 37.7±0.1^{∗∗∗} | 37.6±0.1^{∗∗∗} |
| Positive control group | 8 | 36.7±0.1 | 37.0±0.1## | 37.0±0.1### | 37.0±0.1### | 37.0±0.1### |
| BD-10-10 mg/kg | 8 | 36.7±0.1 | 36.8±0.05### | 37.2±0.1### | 37.4±0.1 | 37.5±0.1 |
| BD-10-50 mg/kg | 8 | 36.6±0.04 | 37.0±0.1## | 37.4±0.04## | 37.4±0.01# | 37.4±0.09## |

| | | | | | | |
|---|---|---|---|---|---|---|
| (Compared with the normal control group, ^{∗∗} P<0.01, ^{∗∗∗} P<0.001; compared with the model group, # P<0.05, ## P<0.01, ### P<0.001) | | | | | | |

Experimental results:
Immediately after oral administration of aspirin (300 mg/kg) and protein BD-10 (10 mg/kg, 50 mg/kg), subcutaneous injection of 20% yeast was performed to establish a model. The animal body temperature was monitored at 2 hours, 4 hours, 6 hours, and 8 hours after modeling. The results show that:
1) The body temperature of rats in the model group increased significantly at 2 hours, 4 hours, 6 hours, and 8 hours after modeling. Compared with the normal group, P<0.05, which was statistically different. The model was successfully established and was stable and reliable.
2) The positive tool drug aspirin group can effectively inhibit the increase in body temperature of model rats at 2 hours, 4 hours, 6 hours, and 8 hours after modeling. Compared with the model group, P<0.05, there is a statistical difference, and the performance of positive tool drugs is relatively stable.
3) Different doses of protein BD-10 can inhibit the increase in body temperature of model rats to varying degrees after modeling, and have a strong effect. At most of the time points after modeling, the body temperature of the model rats can be inhibited, and compared with the model group, P<0.05, which is statistically different.

### Experimental example 3 The pharmacodynamic test of protein BD-10 (Example 4 protein) on convulsive epilepsy in mice caused by the convulsion agent Pilocarpine (PLO)

Animals: male ICR mice;
Drugs: Pilocarpine HCl (PLO, pilocarpine, pilocarpine hydrochloride), Diazepam (diazepam tablets), protein BD-10.
Experiment grouping:
   Model group:
   Diazepam 2mg/kg group;
   Protein BD-10, 50 mg/kg group, 200 mg/kg group.
Method:
   Model preparation and administration:
   The drug was administered once in the afternoon the day before modeling, PLO-225 mg/kg (modeling agent) was injected intraperitoneally 1 hour after the test drug was administered intragastrically on the day of modeling. And positive drug can be administered once 20 minutes before modeling. Observe for 30 minutes after PLO injection.
   Observation indicators: ①Seizure situation: the time of seizures from Grade II to Grade IV; ②the time to death.
   Seizure grade: Refer to Racine grading standard: Grade 0: No response; Grade I: manifested as twitching of facial muscles or the corners of the mouth; Grade II: can nod; Grade III: twitching of one limb; Grade IV: rigidity or body twitching; Grade V: generalized epilepsy (generalized tonic seizures).
Data processing:
   Count the number of Grade IV seizures and deaths in each group of mice in the experiment; Grade II, III and IV incubation period. The incubation period of mice that did not attack to Grade IV was recorded as a maximum of 1800 seconds. Chi-square test was used for statistics of the number of cases. The mean value and standard error of the incubation period were calculated, and TTEST was used to compare the model group with other groups. P<0.05 was considered as a significant difference.

Experimental results: see Table 3 and Table 4.

**Table 3. Experiments of tested drugs on PLO-induced epilepsy in mice-statistics of cases**

| Group | Cases of experiment | Cases of Grade IV | Grade IV seizure rate | Cases of deaths | mortality rate |
|---|---|---|---|---|---|
| Model group | 10 | 8 | 80% | 0 | 0 |
| Diazepam 2 mg/kg | 10 | 0^{∗∗} | 0^{∗∗} | 0 | 0 |
| BD-10-50 mg/kg | 10 | 9 | 90% | 0 | 0 |
| BD-10-200 mg/kg | 10 | 7 | 70% | 0 | 0 |

| | | | | | |
|---|---|---|---|---|---|
| (Compared with the model group, ^{∗}P<0.05, ^{∗∗}P<0.01) | | | | | |

**Table 4. Experiments of tested drugs on PLO-induced epilepsy in mice-Grade II, Grade III and Grade IV seizures incubation period (mean±SEM)**

| Group | Grade II seizure incubation period (s) | Grade III seizure incubation period (s) | Grade IV seizure incubation period (s) |
|---|---|---|---|
| Model group | 86±5 | 142±6 | 894±164 |
| Diazepam 2 mg/kg | 109±8^{∗} | 182±14^{∗} | 1800±0^{∗∗} |
| BD-10-50 mg/kg | 93±4 | 166±8^{∗} | 871±144 |
| BD-10-200 mg/kg | 90±6 | 151±7 | 918±198 |

| | | | |
|---|---|---|---|
| (Compared with the model group, ^{∗}P<0.05, ^{∗∗}P<0.01) | | | |

Experimental results:
1) Experimental results show that the rate of Grade IV seizure in the model group is 80%. None of the 40 mice died.
2) Positive drugs can completely suppress the rate of Grade IV epileptic seizure and significantly prolong the epileptic seizure incubation period of Grade II, III and IV in mice.
3) In the comparison of the incubation period of epilepsy Grade III, the BD-10 50mg/kg dose group was statistically different from the model group.

### Experimental example 4 Efficacy test of protein BD-10 (Example 4 protein) on pentylenetetrazole (PTZ)-induced epilepsy in mice

Animals: male ICR mice;
Medicines: Pentylenetetrazol (PTZ), Retigabine, Protein BD-10.
Experiment grouping:
   Model group;
   Retigabine 60 mg/kg group;
   Protein BD-10, 50 mg/kg group, 200 mg/kg group;
Method:
   Model preparation and administration:
   The drug was administered once in the afternoon the day before modeling, On the day of modeling, PTZ-65mg/kg (modeling agent) was injected intraperitoneal 1 hour after the test drug was administered intragastrically, and the positive drug can be administered once half an hour before modeling. Continue to observe for 15 minutes after injection of PTZ.
   Observation index: ①Seizure situation: the time of seizures from Grade III to Grade VI; ②Death situation
   Seizure grade: Refer to Racine grading standard: Grade 0: No response; Grade I: manifested as twitching of facial muscles or the corners of the mouth; Grade II: can nod; Grade III: twitching of one limb; Grade IV: rigidity or body twitching; Grade V: generalized epilepsy (generalized tonic seizures).
Data processing:
   Count the cases of seizures and deaths in each group of mice in the experiment; Grade III and IV incubation period. The incubation period of mice that have not attacked to Grade IV is recorded as the maximum of 900 seconds. Chi-square test was used for statistics of the number of cases. Calculate the mean and standard error of the incubation period. Use TTEST to compare the model group with other groups, and P<0.05 is considered as a significant difference.

Experimental results: see Table 5 and Table 6.

**Table 5. Test drug on PTZ-induced epilepsy in mice-statistics of cases**

| Group | Cases of experiment | Cases of Grade IV | Grade IV seizure rate | Cases of deaths | mortality rate |
|---|---|---|---|---|---|
| | | | | | |
| Model group | 10 | 9 | 90% | 1 | 10% |
| Retigabine 60 mg/kg | 10 | 2^{∗∗} | 20%^{∗∗} | 0 | 0 |
| BD-10-50 mg/kg | 10 | 8 | 80% | 0 | 0 |
| BD-10-200 mg/kg | 10 | 7 | 70% | 0 | 0 |

| | | | | | |
|---|---|---|---|---|---|
| (Compared with the model group, ^{∗}P<0.05, ^{∗∗}P<0.01) | | | | | |

**Table 6. Experiment of the test drug on PTZ-induced epilepsy in mice-the incubation period of Grade III and IV seizures (mean±SEM)**

| Group | Grade III seizure incubation period (s) | Grade IV seizure incubation period (s) |
|---|---|---|
| Model group | 63±6 | 185±84 |
| Retigabine 60 mg/kg | 83±7^{∗} | 745±103^{∗∗} |
| BD-10-50 mg/kg | 63±2 | 296±106 |
| BD-10-200 mg/kg | 81±9 | 359±120 |

| | | |
|---|---|---|
| (Compared with the model group, ^{∗}P<0.05, ^{∗∗}P<0.01) | | |

Experimental results:
1) The experimental results showed that the rate of Grade IV seizure in the model group was 90%. Two of the 40 mice died.
2) Positive drugs can significantly reduce the rate of Grade IV epileptic seizure, and significantly prolong the incubation period of Grade III and IV seizures in mice.
3) In the comparison of the incubation period of Grade IV epilepsy, the BD-10 has a tendency to extend the incubation period, but due to the large standard error, there is no statistical difference compared with the model group.

### Experimental example 5 The pharmacodynamic test of protein BD-10 (Example 4 protein) on the expectorant of phenol red excretion method in mice.

Animals: male ICR mice;
Drugs and reagents: Mucosolvan (ambroxol hydrochloride tablets), phenol red, sodium bicarbonate, protein BD-10;
Instruments: centrifuge (Sigma-3K15 type), balance (XS105DU type), Microplate tester (BIO-TEK type).
Experiment grouping:
   Solvent control group;
   Mucosolvan 30 mg/kg group;
   Protein BD-10, 20 mg/kg group, 50 mg/kg group.
Method:
   Model preparation and administration:
   The animals were fasted and water was taken freely 16 hours before the experiment. Orally administered Mucosolvan and different doses of protein BD-10 (administration volume 10 ml/kg) in groups, and the solvent control group was given the same volume of distilled water.

One hour later, 2.5% phenol red solution was injected intraperitoneally. Mice were sacrificed by neck dislocation after 30 minutes. Take a trachea from below the thyroid cartilage to the branch of the trachea, and put the trachea into 3 ml 5% NaHCO₃ solution and let it stand for 3 hours.

Take 1 ml of the supernatant and centrifuge at 3000 rpm for 5 minutes. Measure and record the absorbance at 546 nm. According to the standard curve of phenol red, the excretion of phenol red was calculated.

Data processing:
Record the time point of oral administration, the time point of intraperitoneal injection of 2.5% phenol red solution, and the time point of taking the trachea respectively; The absorbance of each group of samples was measured by the microplate reader at 546nm, Calculate the excretion of phenol red according to the standard curve of phenol red. Calculate the mean and standard error of the data in each group, and use TTEST to compare the solvent control group with other groups, and P<0.05 is considered as a significant difference.

Experimental results:
Give Mucosolvan (30 mg/kg) and different doses of protein BD-10 (20 mg/kg, 50 mg/kg). One hour later, 2.5% phenol red solution was intraperitoneally injected, and 30 minutes later, the mice were sacrificed by neck dislocation. Take the trachea from below the thyroid cartilage to the branch of the trachea, put the trachea into 3 ml of 5% NaHCO₃ solution and let it stand for 3 hours, take 1ml of supernatant, centrifuge at 3000rpm for 5 minutes, measure and record the absorbance at 546 nm. According to the standard curve of phenol red, the excretion of phenol red was calculated. The results are shown in Table 7.

**Table 7. The effect of the test drug on the expectorant effect of the phenol red excretion method in mice (X±SEM)**

| Group | N | Phenol red excretion µg/ml) | P |
|---|---|---|---|
| Solvent control group | 10 | 0.506±0.040 | --- |
| Mucosolvan 30 mg/kg | 10 | 1.061±0.117^{∗∗} | 0.001 |
| BD-10-20 mg/kg | 10 | 0.746±0.035^{∗∗} | 0.001 |
| BD-10-50 mg/kg | 10 | 0.668±0.063^{∗} | 0.042 |

| | | | |
|---|---|---|---|
| (Compared with the solvent control group, ^{∗} P<0.05, ^{∗∗} P<0.01) | | | |

Experimental results:
1) The experimental results showed that compared with the solvent control group, the amount of phenol red excretion in the Mucosolvan 30 mg/kg group was significantly increased, P<0.05, which was statistically significant.
2) Compared with the solvent control group, the BD-10 20mg/kg and 50mg/kg dose groups significantly increased the excretion of phenol red, P<0.05, which was statistically significant.

### Experimental example 6 The effect of protein BD-10 (Example 4 protein) on the antitussive effect of the cough induced by ammonia water in mice.

Animals: male ICR mice;
Drugs and reagents: dextromethorphan hydrobromide, ammonia water, 0.2% CMC-Na, protein BD-10;
Apparatus: Compressed nebulizer (403T type), balance (XS105DU type).
Experiment grouping:
Solvent control group;
Dextromethorphan 15 mg/kg group;
Protein BD-10, 20 mg/kg group, 50 mg/kg group.

Method:
Model preparation and administration:
Dextromethorphan and different doses of protein BD-10 (administration volume 10ml/kg) were given orally in groups, and the solvent control group was given the same volume of distilled water. One hour later, mice were put into a sealed box and atomized 10% ammonia water for 10 seconds, and then observed and recorded the incubation period of cough in mice and the number of coughs in 2 minutes.
Data processing:
Record the time point of oral administration, the time point of atomization experiment, the incubation period of mice cough and the number of coughs within 2 minutes, respectively. The incubation period of cough refers to the number of seconds from the start of the atomization of ammonia to the occurrence of cough. The performance of coughing in mice is based on contraction of their abdominal muscles (breast contraction) and opening their mouths at the same time. Calculate the mean and standard error of each group of data, and use TTEST to compare the model group with other groups, and P<0.05 is considered as a significant difference.
Experimental results:
Give dextromethorphan (15 mg/kg) and different doses of protein BD-10 (20 mg/kg, 50 mg/kg) in advance, One hour later, the mice were put into a sealed box and atomized 10% ammonia water for 10 seconds, and then the mice were observed and recorded the incubation period of coughing and the number of coughs within 2 minutes. The results are shown in Table 8.

**Table 8. Antitussive effect experiment of tested drugs on mice cough induced by ammonia water (X±SEM)**

| Group | N | Incubation period (s) | P | Number of coughs | P |
|---|---|---|---|---|---|
| Solvent control group | 9 | 26.9±2.3 | --- | 67.1±5.7 | --- |
| Dextromethorphan 15 mg/kg | 9 | 38.4±4.5^{∗} | 0.037 | 34.0±2.9^{∗∗} | 0.001 |
| BD-10-20 mg/kg | 9 | 31.4±2.6 | 0.206 | 55.3±4.2 | 0.114 |
| BD-10-50 mg/kg | 9 | 29.4±3.0 | 0.505 | 59.8±3.9 | 0.303 |

| | | | | | |
|---|---|---|---|---|---|
| (Compared with the solvent control group, ^{∗} P<0.05, ^{∗∗} P<0.01) | | | | | |

Experimental results:
1) The experimental results showed that the dextromethorphan group had a significant improvement in the incubation period and the number of coughs compared with the solvent control group, P<0.05, which was statistically significant.
2) BD-10 has a tendency to prolong the incubation period and reduce the number of coughs, but there is no statistically significant.

### Experimental example 7 The pharmacodynamic test of protein BD-10 (Example 4 protein) on acetic acid writhing in ICR mice.

Animals: male ICR mice;
Drugs and reagents: aspirin, physiological saline, glacial acetic acid, protein BD-10.
Experiment grouping:
Model group;
Aspirin 300 mg/kg group;
Protein BD-10, 50 mg/kg group, 200 mg/kg group.

method:
One day after the experimental animals adapt to the environment, Aspirin 300 mg/kg, protein BD-10 50 mg/kg, 200 mg/kg were given orally one hour in advance, and the administration volume was 10 ml/kg; Then, 0.6% acetic acid solution was injected intraperitoneally, and the incubation period (seconds) and frequency of writhing in the animal was observed within 15 minutes.
Data processing:
Calculate the mean and standard error of the data in each group. Compared with the model group by TTEST, P<0.05 was considered as statistically different.
Experimental results:
One hour after oral administration of aspirin 300 mg/kg and different doses of protein BD-10 (50 mg/kg, 200 mg/kg), 0.6% acetic acid solution was intraperitoneally injected to observe the writhing incubation period and frequency of ICR mice. The results are shown in Table 9.

**Table 9. The effects of the tested drugs on the acetic acid writhing test of ICR mice**

| Group | N | Weight (g) | Writhing incubation period (seconds) | Frequency of writhing (times) |
|---|---|---|---|---|
| Model group 0.6% acetic acid | 18 | 22.8±0.2 | 204.2±19.6 | 33.3±2.5 |
| aspirin 300 mg/kg | 13 | 23.2±0.2 | 290.3±37.0^{∗} | 15.2±2.9^{∗∗∗} |
| BD-10-50 mg/kg | 13 | 24.1±0.3 | 237.4±44.8 | 22.7+3.9^{∗} |
| BD-10-200 mg/kg | 13 | 23.7±0.4 | 251.2±43.3 | 19.8±3.7 ^{∗∗} |

| | | | | |
|---|---|---|---|---|
| (Compared with the model group, ^{∗∗} P<0.01) | | | | |

Experimental results:
0.6% acetic acid solution was injected into the abdominal cavity of mice, which caused deep and large area and long-term painful stimulation, causing the mice to writhe (the abdomen was contracted into an "S" shape, the trunk and hind legs were stretched, the buttocks were raised and creeping). The incubation time to start writhing and times of writhing in mice were used as the pain response indexs to determine whether the test sample had analgesic effect. The results of this experiment show:
1) Aspirin 300 mg/kg can significantly delay the incubation period of writhing and reduce the times of writhing, and has a certain analgesic effect. Compared with the model group, P<0.05, which is statistically significant.
2) BD-10 50mg/kg and 200mg/kg dose groups can significantly reduce the number of writhing mice. Compared with the model group, P<0.05, which is statistically significant.

## Claims

1. A keratin BD-10, wherein the amino acid sequence of the keratin BD-10 is:
(1) the amino acid sequence set forth in SEQ ID NO:1 in the sequence listing;
(2) an amino acid sequence that basically maintains the same biological function formed by substitution, deletion or addition of 1-35 amino acids to the amino acid sequence set forth in SEQ ID NO: 1 in the sequence listing.

2. The keratin BD-10 of claim 1, wherein the keratin BD-10 may have a conventional modification; or the keratin BD-10 is further linked with a tag for detection or purification.

3. The keratin BD-10 of claim 2, wherein the conventional modification includes acetylation, amidation, cyclization, glycosylation, phosphorylation, alkylation, biotinylation, fluorescent group modification, polyethylene glycol PEG modification, immobilization modification, sulfation, oxidation, methylation, deamination, formation of disulfide bond or breakage of disulfide bond; the tag includes His6, GST, EGFP, MBP, Nus, HA, IgG, FLAG, c-Myc, Profinity eXact.

4. A nucleic acid molecule encoding the keratin BD-10 of any one of claims 1-3.

5. The nucleic acid molecule of claim 4, wherein the nucleotide sequence of the nucleic acid molecule is:
(1) the nucleotide sequence set forth in SEQ ID NO:2 in the sequence listing;
(2) a nucleotide sequence obtained by sequence optimization based on the nucleotide sequence set forth in SEQ ID NO:2;
(3) a nucleotide sequence complementary to the nucleotide sequence in (1) or (2) above.

6. An expression vector, wherein the expression vector contains the nucleic acid molecule of claim 4 or 5.

7. A host cell, wherein the host cell contains the expression vector of claim 6 or the nucleic acid molecule of claim 4 or 5 integrated into the genome.

8. The host cell of claim 7, wherein the host cell includes bacteria, yeast, Aspergillus, plant cells, or insect cells.

9. The host cell of claim 8, wherein the bacteria includes *Escherichia coli.*

10. A method for preparing the keratin BD-10 of any one of claims 1-3, wherein the method comprises the following steps:
A. synthesizing a nucleic acid molecule corresponding to the keratin BD-10 of any one of claims 1-3, linking the nucleic acid molecule to a corresponding expression vector, transforming the expression vector into a host cell, and culturing the host cell with the expression vector in a fermentation device under certain conditions and inducing the expression of the keratin BD-10 to obtain a crude protein solution containing the keratin BD-10;
B. subjecting the crude protein solution obtained in step A to separation and purification, and drying to obtain the keratin BD-10.

11. The method of claim 10, wherein in step A, the host cell is mainly selected from *Escherichia coli,* the keratin BD-10 is expressed in the inclusion bodies of *Escherichia coli,* and the fermentation device includes shake flask or fermenter.

12. The method of claim 10, wherein in step A, after inducing the expression of the keratin BD-10, impurities in which may be removed with a cleaning agent to obtain the crude protein solution by dissolving in a solution.

13. The method of claim 10, wherein in step B, the separation and purification method includes ultrafiltration microfiltration membrane technology purification method, column chromatography purification method, salting out method, and dialysis method.

14. A pharmaceutical composition, wherein the pharmaceutical composition contains the keratin BD-10 of any one of claims 1-3 and a pharmaceutically acceptable carrier or excipient.

15. The use of the keratin BD-10 of any one of claims 1-3 or the nucleic acid molecule of claim 4 or 5 or the expression vector of claim 6 or the host cell of any one of claims 7-9 or the pharmaceutical composition of claim 14 in the preparation of medicament for antipyretic, analgesic, antitussive, expectorant, anticonvulsant, antiepileptic, anti-hypertension, anti-inflammatory, and antiviral.
